# EUROPEAN PATENT SPECIFICATION

(11) **EP 3 187 297 B1**
(45) Date of publication and mention of the grant of the patent: **04.12.2019**
(21) Application number: 16397541.0
(22) Date of filing: 28.12.2016
(51) Int. Cl.: B23K 26/20, B23K 26/57, A61N 1/375

(54) **HERMETIC ELECTRICAL FEEDTHROUGH WITH SUBSTRATE PIECE AND A METHOD FOR IMPLEMENTING IT**
HERMETISCHE ELEKTRISCHE DURCHFÜHRUNG MIT SUBSTRATSTÜCK UND VERFAHREN ZUR IHRER AUSFÜHRUNG
PASSAGE ÉLECTRIQUE HERMETIQUE AVEC PIÈCE DE SUPPORT ET PROCÉDÉ DE MISE EN OEUVRE

(30) Priority: 31.12.2015 FI 20156041
(43) Date of publication of application: 05.07.2017
(73) Proprietor: Schott Primoceler Oy, 33720 Tampere (FI)
(72) Inventor: Määttänen, Antti, 33720 Tampere (FI)
(74) Representative: Berggren Oy, Tampere

(56) References cited:
- WO-A1-97/06853
- US-A1- 2014 231 133

## Description

The invention relates to at least one hermetic electrical feedthrough in a substrate piece, and a method for implementing it, said piece comprising at least two substrate layers, a conductor layer fitted between these substrate layers, a component fitted inside said piece and hermetically isolated from the exterior, and a conductor formed of the conductor layer and extending from the outer surface of said piece to the component for providing an electrical connection, the feedthrough being fitted between said substrate layers. The invention also relates to a method for implementing the hermetic electrical feedthrough in the substrate piece.

Uses of the invention include various substrate pieces, which comprise optoelectronic components, and substrate pieces, which comprise mobile device sensors. What is common for said pieces is that they can be composed of substrate layers and a conductor layer by cementing, soldering or welding these layers together such that the component inside the piece becomes hermetically isolated from the exterior.

Said pieces are transparent or partially transparent, and when a focused laser beam is used for joining their layers, a layer through which the laser beam is transmitted to the point of welding is transparent at the wavelength of the laser beam used. Said pieces are known to comprise a conductor layer fitted between the substrate layers, wherein at least one electrically conductive conductor is formed of the conductor layer, and wherein the conductor extends from said internal component to the outer surface of the substrate piece for transferring power to the component and for transmitting information to the component or from the component to the outside of the substrate piece. Metals and their compounds are generally used as the materials for the conductor layers.

One known solution for forming a conductor layer is to place a thin ITO film (Indium Tin Oxide) between the substrate layers. As examples of substrate layers may be mentioned glass, sapphire and/or silicon substrate semiconductor disks which are used in microelectronics. Thus, hermetic feedthroughs of electrical buses may be formed in substrate pieces by using the solution according to the invention.

It is known to make the above-mentioned substrate pieces, for example, by fitting a conductor layer between two substrate layers placed against each other and by cementing or soldering the layers together into one piece. Another way is to cement or solder the conductor layer to two substrate layers and to then cement or solder these conductor layers together. The conductor layer is fitted in place such that at least one conductor between the component and the outer surface of the substrate piece may be formed via the conductor layer.

As a greatest disadvantage of above-mentioned prior art may be considered the fact that it is very difficult to make completely hermetic connections by cementing and soldering. These operations require heating of the parts of the substrate piece and cause warping of the layers, wherein the connecting surfaces become non-planar or otherwise uneven due to the warping, and the joints do not become hermetically sealed. Attempts have been made to lessen this disadvantage by using high material layer thicknesses, which in turn considerably increases material costs. Thermal energy stored in the layers also causes bubbling in the conductor layer material, which is thinner when compared with the other layers, which in turn eliminates the advantage brought by increasing the material thickness of the substrate layers, and the unreliability of the seams is thus not eliminated.

Another prior art method for forming electrical feedthroughs in substrate pieces is to assemble the piece, for example, of two substrate layers by welding these layers together by laser welding, i.e. to form a hermetic peripheral weld around the component, between the component and the outer surfaces of the piece, in the common interface of the layers, and to form a feedthrough through the wall and fill this feedthrough extending to the component with a metal (TGV technique).

The greatest disadvantages of this method may be considered to be the high manufacturing costs resulting from several different working phases, and the unreliable final result. It is difficult to make the feedthrough completely gas-tight.

Achieving hermetic sealing for the uses of the invention is very important because the access of oxygen and impurities into the component is very detrimental. In practice, it has been found that even a very small gap in the cement, solder or drilled/cut feedthrough may cause a leakage of gas into the interior of the substrate piece.

The prior art techniques described above are widely used in the manufacture of substrate pieces.

Using a 360° peripheral weld, as described above, for producing a substrate piece containing a conductor layer has not been a practical method because it destroys the feedthrough points of the conductor layer.

WO9706853 discloses forming a hermetically sealed feedthrough by depositing a pattern of conductive material on the upper surface of a first insulating layer, and by depositing a second insulating layer over the conductive material. WO9706853 discloses bonding a frame to the second insulating layer by diffusion bonding, and laser welding a lid to the frame.

A purpose of the present invention is to provide at least one such hermetic electrical feedthrough in a substrate piece, and a method for implementing it, which avoids the disadvantages of the prior art. The solution according to the invention is characterized by what is disclosed in the characterizing parts of claims 1 and 9.

The greatest advantage of using the invention may be considered to be the very simple, economical and reliable way of producing hermetic feedthroughs for substrate pieces. Thin conductor layers, optimal material thicknesses in the substrate layers and a sophisticated laser technique are utilized in the method according to the invention.

In this document, the term conductor layer refers to a layer whose extent is greater than or equal to the combined extent of the conductors, and the term feedthrough refers to a structure with an opening and a conductor passing through this opening.

The invention is described in greater detail in the accompanying drawings, in which
Fig. 1 shows a view of an assembly for producing a substrate piece as seen directly from a side,
Fig. 2 shows said assembly as seen directly from above,
Fig. 3 shows an enlarged view of area A of Fig. 2,
Fig. 4 shows section B-B of Fig. 3,
Fig. 5 shows section C-C of Fig. 3,
Fig. 6 shows another embodiment of the invention.

Next, the structure of a preferred application of the invention and a method for implementing it are described, with reference to the above-mentioned drawings.

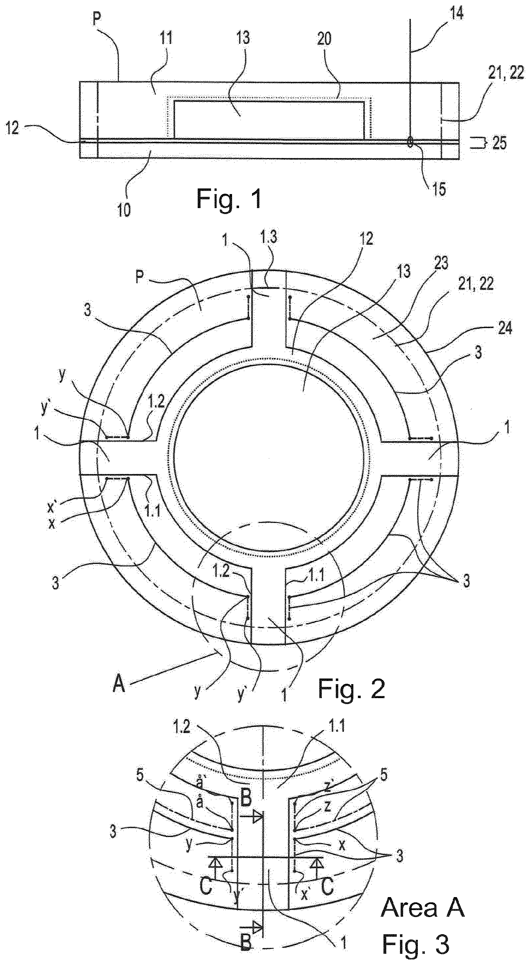

Figs. 1 to 3 show an assembly where a substrate piece P is made of two substrate layers, such as a first substrate layer 10 and a second substrate layer 11. In this example, the material of these layers is glass and the layers are placed on top of each another and between them is fitted a conductor layer 12. Fitting the conductor layer between the substrate layers 10, 11 may be performed e.g. by fixing a conductor film on one or both of the substrate layers, and when said substrate layers are placed against one another such that the film/films remain between them, the conductor layer 12 is formed. An interior 20 is formed in the substrate layers, and a component 13 is fitted in this space, wherein the component 13 is an optical component in this example. The conductor layer 12 is of electrically conductive material and it is a so-called ITO film in this example. In this example, the conductor layer 12 comprises four projecting parts extending to the outer edge 24 of the assembly at a 90° pitch and its central part is connected to the component 13. The layers of the assembly, the first and second substrate layer 10, 11, and the conductor layer 12 between them are pressed tightly together. In this example, the material thickness c of the conductor layer 12 is 300 nm, and a focused focal point 15 of the laser beam 14 is directed to the height level of
a common interface area 25 of said three layers, such that it meets the point x shown in Fig. 2, wherein the materials of said substrate layers melt and are mixed at this point and in its immediate vicinity. In this example, a point x is substantially at the first edge 1.1. of the conductor being formed. The laser beam 14 is moved with respect to the substrate piece P being formed such that it proceeds according to the shape of the circular periphery 21 of the substrate piece, inside this periphery and outside the conductor layer 12, substantially to a point y at the second edge 1.2 of the next conductor 1. Thus, a hermetic weld 3 is formed between points x and y. In the substrate piece P produced in this example are formed four conductors 1 of the projecting parts of the conductor layer 12, wherein the orientation of each projecting part is substantially parallel with a radius of the circular periphery 21 of said piece and wherein the projecting parts are evenly distributed, i.e. at 90° intervals, wherein a weld 3 of the type described above is formed between each pair of adjacent conductors. After this, material is cut off from the edge of the substrate layers outside the welds 3 such that said circular periphery 21 of the substrate piece is formed at the cutting point 22.

Said substrate layers 10, 11 may be cut into any shape and also so as to be of different extents, whereby the ends 1.3 of the conductors 1 will protrude on the surface of one or the other substrate layer on the outer edge of the substrate piece P, and it is easy to connect them to a power and/or data network outside the piece. A substrate layer, or both of them, may also be cut in advance into their final size, which may be the same or different.

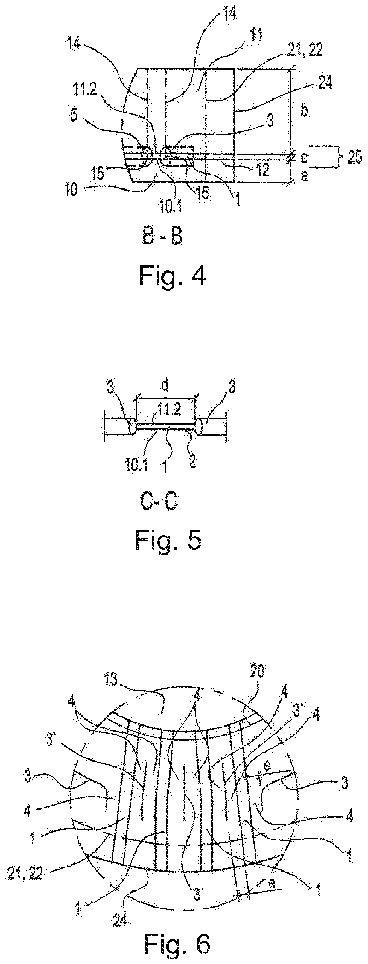

Figs. 3 to 5 show in greater detail the situation achieved at the feedthrough point. An opening 2 having the size of the cross-section of the conductor 1 has been formed around the conductor 1, wherein there is a weld 3 is on both sides of the opening 2 (Fig. 5). When the weld 3 formed cools down, the change of temperature creates thermal tensions in the substrate layers 10,11 wherein the thermal tensions constrict the opposite surfaces of the first and second substrate layer towards each other, i.e. against the conductor 1, and in this way an opening 2 having substantially the extent of the cross-section of the conductor 1 is formed, and as a result four hermetic feedthroughs of the conductor 1 are formed in the substrate piece (P).

In the structure according to the example described above, there are hermetic joints in the feedthrough between the conductor 1 and the mutual connecting surfaces of the substrate layers 10, 11 connecting to it, which thus renders the feedthrough hermetic throughout.

Practical tests have shown that an electrical feedthrough formed in the above manner is completely hermetic. No leakage points remain between the conductor 1 and the opening 2, through which leakage points even capillary forces could convey gas. Thus, the feedthrough formed is surrounded by the first surface 10.1 of the first substrate piece 10 and by the second surface 11.2 of the second substrate piece on two opposite sides, and by a weld 3 on the two other opposite sides.

Figs. 2 to 4 further show an application of the invention, where the ends of the weld 3 continue from points x and y such that the orientation of the ends is substantially parallel with the first and second edge 1.1, 1.2 of the conductor, towards the periphery 21 of the substrate piece P, from point x to point x' and from point y to point y' (broken lines). By using this procedure, the opening 2 and the feedthrough of the conductor 1 may be made longer (deeper), thus further ensuring its leak-tightness. In practice, this type of weld 3 is formed starting from point x' or y' and extending without a break to its end point y'/x'. Point x' and/or y' may also be located outside the periphery 21. The weld may also be continued from the area of the second substrate piece being formed to the piece at hand or vice versa, for example, when the blanks of these pieces are adjacent to one another during a manufacturing phase.

One embodiment of the invention is implemented, as shown in Figs. 3 and 4, such that adjacent to the weld 3 is formed another weld 5 (dot-and-dash line in Fig. 3). In this example, the weld 3 also includes the parts between points x-x' and y-y', as mentioned above, and in the second weld 5 are formed corresponding parts between points z-z' and å-å' proceeding in the opposite direction with respect to the above. By means of this procedure, the depth of the feedthrough may be further increased.

In the method according to the invention may be used, for example, the following thicknesses of material layers:
- first substrate layer, a, =>30 µm,
- second substrate layer, b, =>30 µm,
- conductor layer c, 1-600 nm,
and in the feedthrough area, for example, the following dimensions:
- conductor 1 width d, =>1-500 µm,
- zone 4 width e, 0-200 nm (zone defined below)
- distances x- x', y- y', z- z' ja å- å', 0- 500 µm.

The given dimensions do not, however, limit the use of the invention to these only, but instead these characteristics may, in each specific case, be given any practical values which implement the hermetic feature and sufficient power and/or data transfer capacity.

The invention may also be applied such that the extent of the conductor layer 12 is substantially equal to the extent of one or both of the substrate layers 10, 11, in which case the conductor layer is destroyed at the weld 3 or welds 3, 5 and the conductor 1 is formed between the welds.

Fig. 6 shows an application of the invention where several feedthroughs are positioned close to one another such that they together have a fan-shaped general appearance. In this case, there is one weld 3 in the substrate piece P which is significantly longer than the other welds, almost completely encircling the component, and shorter welds 3' between the feedthroughs. This structure further comprises zones 4 between the welds 3, 3' and the conductors 1, to which said welds do not reach. The width e of these zones is of the order of at most 200 nm and thus, according to empirical knowledge, they also become hermetic, and thus the feedthrough according to this application is also hermetic. The zones 4 may be applied even more widely when using the invention than what is disclosed here. In the zones 4, the joints between the layers are thus not welded together and they are tight also without welding due to their microscopic size.

Part P, which has a feedthrough according to the invention, as well as component 13, may be of various shapes and sizes. No size or geometric shape is excluded when using the invention.

The substrate layers 10, 11 may be of a wide range of materials or combinations of materials, where melting and resolidification may take place by means of a focused laser beam. They may be homogeneous or consist of areas and/or layers of different materials. A film or sheet made of gold or other electrically conductive metal or metallic compound may also be used as the conductor layer 12 instead of said ITO film.

It should be noted that although this specification has concentrated on one type of a preferable implementation example of the invention, the aim is not to limit the use of the invention to concern only examples of this type, but various modifications are possible within the scope of the inventive idea defined in the claims.

## Claims

1. A combination of a substrate piece (P) and a component (13) fitted inside the substrate piece (P), the component (13) being hermetically isolated from the exterior of said piece (P), the piece comprising:
a. at least two substrate layers (10, 11),
b. a conductor layer (12) fitted between said substrate layers,
c. a component (13) fitted inside the substrate piece (P), the component (13) being hermetically isolated from the exterior of said piece,
d. a first conductor (1) made of the conductor layer (12), the first conductor (1) extending from the periphery (21) of said piece to the component (13) for providing an electrical connection,
and wherein a hermetic feedthrough comprising the first conductor (1) is fitted between the substrate layers (10, 11),
**characterized in that**
e. the substrate layers (10, 11) and the conductor layer (12) have a common interface region (25), the substrate layers (10, 11) and the conductor layer (12) are pressed together and joined with each other by means of at least one weld (3) made with a focused laser beam (14), wherein the at least one weld (3) encircles the component (13) at the height level of the common interface region (25), and wherein each weld extends substantially from a first edge (1.1) of a conductor (1) to its second edge or to a second edge (1.2) of another conductor adjacent to it, such that said hermetic feedthrough comprises a zone (4), which is located between the first conductor (1) and a weld (3,3'), wherein joints located within said zone (4) between the layers are not welded together,
f. opposite surfaces of the substrate layers (10, 11) facing towards the conductor (1), a first surface (10.1) and a second surface (11.2), are constricted against the conductor (1) due to thermal tensions caused by welding heat, wherein the joints between the conductor (1) and said surfaces, as well as all other joints remaining in the area between adjacent welds (3, 3'), are hermetic.

2. The combination according to claim 1, **characterized in that** the width (e) of said zone (4) is smaller than or equal to 200 nm.

3. The combination according to claim 1 or 2, **characterized in that** a second weld (5) has been formed adjacent to a weld (3).

4. The combination according to claim 1, 2 or 3, **characterized in that** the ends of a weld (3) and/or the ends of a second weld (5) have been arranged to be substantially parallel with the first and second edge (1.1, 1.2) of the conductor (1).

5. The combination according to any of the claims 1 to 4, **characterized in that** the conductor layer (12) is substantially of the extent of the first and/or second substrate layer (10, 11).

6. The combination according to any of the claims 1 to 4, **characterized in that** the conductor layer (12) has been shaped before fitting in place such that only parts forming the conductors (1) extend to the periphery (21) of the substrate piece (P).

7. The combination according to any of the claims 1 to 6, **characterized in that** the material of the first and/or second substrate layer (10, 11) is glass, silicon, sapphire or a combination material of these.

8. The combination according to any of the claims 1 to 7, **characterized in that** the material of the conductor layer (12) and the conductor (1) is ITO or an electrically conductive metal/metal compound.

9. A method for implementing a hermetic electrical feedthrough in a substrate piece (P), wherein said piece is formed as follows:
a. a first and a second substrate layer (10, 11) are placed against each other after a component (13) has been fitted in a space (20) between said substrate layers and after a conductor layer (12) has been placed between said layers,
b. a hermetic feedthrough comprising a first conductor (1) is fitted between the substrate layers (10, 11), the first conductor (1) is formed of the conductor layer (12), and wherein the first conductor (1) extends from the periphery (21) of the substrate piece (P) to the component (13) for providing an electrical connection from outside the substrate piece (P) to the component (13), **characterized in that**
c. the substrate layers (10, 11) and the conductor layer (12) have a common interface region (25), the substrate layers (10, 11) and the conductor layer (12) between them are joined together by means of at least one weld (3, 3') made with a focused laser beam (14), wherein the at least one weld (3, 3') encircles the component (13) at the height level of the common interface area (25), wherein each weld extends substantially from a first edge (1.1) of a conductor (1) to its second edge or to a second edge (1.2) of another conductor adjacent to it, such that said hermetic feedthrough comprises a zone (4), which is located between the first conductor (1) and a weld (3,3'), wherein joints located within said zone (4) between the layers are not welded together,
d. opposite surfaces of the substrate layers (10, 11) facing towards the conductor (1), a first surface (10.1) and a second surface (11.2), are constricted against the first conductor (1) after welding due to thermal tensions caused by welding heat, wherein joints between the first conductor (1) and said surfaces, as well as all other joints remaining in the area between adjacent welds (3, 3'), are hermetic.

10. The method according to claim 9, **characterized in that** a second weld (5) is formed adjacent to a first weld (3), and wherein ends of said welds (3,5) are substantially parallel with the first/second edges (1.1, 1.2) of the first conductor (1).

## Patentansprüche

1. Kombination aus einem Substratstück (P) und einer in dem Substratstück (P) eingepassten Komponente (13), wobei die Komponente (13) hermetisch von dem Äußeren des Stücks (P) isoliert ist, wobei das Stück umfasst:
a. mindestens zwei Substratschichten (10, 11),
b. eine Leiterschicht (12), die zwischen den Substratschichten eingepasst ist,
c. eine Komponente (13), die in dem Substratstück (P) eingepasst ist, wobei die Komponente (13) hermetisch von dem Äußeren des Stücks isoliert ist,
d. einen ersten Leiter (1), der aus der Leiterschicht (12) gefertigt ist, wobei sich der erste Leiter (1) von dem Umfang (21) des Stücks zur Komponente (13) erstreckt, um eine elektrische Verbindung bereitzustellen, und wobei eine hermetische Durchführung, die den ersten Leiter (1) umfasst, zwischen den Substratschichten (10, 11) eingepasst ist, **dadurch gekennzeichnet, dass**
e. die Substratschichten (10, 11) und die Leiterschicht (12) einen gemeinsamen Grenzflächenbereich (25) aufweisen, wobei die Substratschichten (10, 11) und die Leiterschicht (12) zusammengepresst und mittels mindestens einer Schweißstelle (3), die mit einem fokussierten Laserstrahl (14) hergestellt ist, zusammengefügt sind, wobei die mindestens eine Schweißstelle (3) die Komponente (13) auf der Höhe des gemeinsamen Grenzflächenbereichs (25) umgibt, und wobei sich jede Schweißstelle im Wesentlichen von einer ersten Kante (1.1) eines Leiters (1) zu seiner zweiten Kante oder zu einer zweiten Kante (1.2) eines weiteren, an diesen angrenzenden Leiters erstreckt, sodass die hermetische Durchführung eine Zone (4) umfasst, die sich zwischen dem ersten Leiter (1) und einer Schweißstelle (3, 3') befindet, wobei Verbindungsstellen, die sich innerhalb der Zone (4) zwischen den Schichten befinden, nicht verschweißt sind,
f. gegenüberliegende Oberflächen der Substratschichten (10, 11), die zum Leiter (1) weisen, eine erste Oberfläche (10.1) und eine zweite Oberfläche (11.2), durch von der Schweißwärme verursachte Wärmespannungen gegen den Leiter (1) verengt werden, wobei die Verbindungsstellen zwischen dem Leiter (1) und den Oberflächen sowie alle anderen Verbindungsstellen, die in dem Bereich zwischen angrenzenden Schweißstellen (3, 3') verbleiben, hermetisch sind.

2. Kombination gemäß Anspruch 1, **dadurch gekennzeichnet, dass** die Breite (e) der Zone (4) kleiner oder gleich 200 nm ist.

3. Kombination gemäß Anspruch 1 oder 2, **dadurch gekennzeichnet, dass** eine zweite Schweißstelle (5) angrenzend an eine Schweißstelle (3) gebildet worden ist.

4. Kombination gemäß Anspruch 1, 2 oder 3, **dadurch gekennzeichnet, dass** die Enden einer Schweißstelle (3) und/oder die Enden einer zweiten Schweißstelle (5) so angeordnet worden sind, dass sie im Wesentlichen parallel zur ersten und zweiten Kante (1.1, 1.2) des Leiters (1) sind.

5. Kombination gemäß einem der Ansprüche 1 bis 4, **dadurch gekennzeichnet, dass** die Leiterschicht (12) im Wesentlichen der Erstreckung der ersten und/oder der zweiten Substratschicht (10,11) entspricht.

6. Kombination gemäß einem der Ansprüche 1 bis 4, **dadurch gekennzeichnet, dass** die Leiterschicht (12) geformt worden ist, bevor sie an ihren Platz eingepasst wurde, sodass sich nur Teile, die die Leiter (1) bilden, zum Umfang (21) des Substratstücks (P) erstrecken.

7. Kombination gemäß einem der Ansprüche 1 bis 6, **dadurch gekennzeichnet, dass** das Material der ersten und/oder der zweiten Substratschicht (10, 11) Glas, Silicium, Saphir oder ein Kombinationsmaterial aus diesen ist.

8. Kombination gemäß einem der Ansprüche 1 bis 7, **dadurch gekennzeichnet, dass** das Material der Leiterschicht (12) und des Leiters (1) ITO oder ein(e) elektrisch leitende(s) Metall/Metallverbindung ist.

9. Verfahren zum Implementieren einer hermetischen elektrischen Durchführung in einem Substratstück (P), wobei das Stück wie folgt gebildet wird:
a. eine erste und eine zweite Substratschicht (10, 11) werden aneinander angelegt, nachdem eine Komponente (13) in einen Raum (20) zwischen den Substratschichten eingepasst worden ist und nachdem eine Leiterschicht (12) zwischen den Schichten platziert worden ist,
b. eine hermetische Durchführung, die einen ersten Leiter (1) umfasst, wird zwischen den Substratschichten (10, 11) eingepasst, der erste Leiter (1) wird aus der Leiterschicht (12) gebildet, und wobei sich der erste Leiter (1) vom Umfang (21) des Substratstücks (P) zur Komponente (13) erstreckt, um eine elektrische Verbindung von außerhalb des Substratstücks (P) zur Komponente (13) bereitzustellen, **dadurch gekennzeichnet, dass**
c. die Substratschichten (10, 11) und die Leiterschicht (12) einen gemeinsamen Grenzflächenbereich (25) aufweisen, wobei die Substratschichten (10, 11) und die Leiterschicht (12) zwischen diesen mittels mindestens einer Schweißstelle (3, 3'), die mit einem fokussierten Laserstrahl (14) hergestellt wird, zusammengefügt werden, wobei die mindestens eine Schweißstelle (3, 3') die Komponente (13) auf der Höhe des gemeinsamen Grenzflächenbereichs (25) umgibt, wobei sich jede Schweißstelle im Wesentlichen von einer ersten Kante (1.1) eines Leiters (1) zu seiner zweiten Kante oder zu einer zweiten Kante (1.2) eines weiteren, an diesen angrenzenden Leiters erstreckt, sodass die hermetische Durchführung eine Zone (4) umfasst, die sich zwischen dem ersten Leiter (1) und einer Schweißstelle (3, 3') befindet, wobei Verbindungsstellen, die sich innerhalb der Zone (4) zwischen den Schichten befinden, nicht verschweißt werden,
d. gegenüberliegende Oberflächen der Substratschichten (10, 11), die zum Leiter (1) weisen, eine erste Oberfläche (10.1) und eine zweite Oberfläche (11.2), durch von der Schweißwärme verursachte Wärmespannungen nach dem Schweißen gegen den ersten Leiter (1) verengt werden, wobei Verbindungsstellen zwischen dem ersten Leiter (1) und den Oberflächen sowie alle anderen Verbindungsstellen, die in dem Bereich zwischen angrenzenden Schweißstellen (3, 3') verbleiben, hermetisch sind.

10. Verfahren gemäß Anspruch 9, **dadurch gekennzeichnet, dass** eine zweite Schweißstelle (5) neben einer ersten Schweißstelle (3) gebildet wird, und wobei Enden der Schweißstellen (3, 5) im Wesentlichen parallel zu der ersten/zweiten Kante (1.1, 1.2) des ersten Leiters (1) sind.

## Revendications

1. Combinaison d'une pièce de substrat (P) et d'un composant (13) ajusté à l'intérieur de la pièce de substrat (P), le composant (13) étant hermétiquement isolé de l'extérieur de ladite pièce (P), la pièce comprenant :
a. au moins deux couches de substrat (10, 11),
b. une couche conductrice (12) ajustée entre lesdites couches de substrat,
c. un composant (13) ajusté à l'intérieur de la pièce de substrat (P), le composant (13) étant hermétiquement isolé de l'extérieur de ladite pièce,
d. un premier conducteur (1) constitué de la couche conductrice (12), le premier conducteur (1) s'étendant à partir de la périphérie (21) de ladite pièce jusqu'au composant (13) pour fournir une connexion électrique,
et dans laquelle une traversée hermétique comprenant le premier conducteur (1) est ajustée entre les couches de substrat (10, 11), **caractérisée en ce que**
e. les couches de substrat (10, 11) et la couche conductrice (12) ont une région d'interface commune (25), les couches de substrat (10, 11) et la couche conductrice (12) sont pressées ensemble et assemblées au moyen d'au moins une soudure (3) réalisée avec un faisceau laser focalisé (14), dans laquelle l'au moins une soudure (3) entoure le composant (13) au niveau de la hauteur de la région d'interface commune (25), et dans laquelle chaque soudure s'étend essentiellement d'un premier bord (1.1) d'un conducteur (1) à son deuxième bord ou à un deuxième bord (1.2) d'un autre conducteur adjacent à celui-ci, de sorte que ladite traversée hermétique comprend une zone (4), qui est située entre le premier conducteur (1) et une soudure (3, 3'), dans laquelle des joints situés dans ladite zone (4) entre les couches ne sont pas soudés ensemble,
f. des surfaces opposées des couches de substrat (10, 11) tournées vers le conducteur (1), une première surface (10.1) et une deuxième surface (11.2), sont resserrées sur le conducteur (1) en raison de tensions thermiques provoquées par la chaleur de soudage, dans laquelle les joints entre le conducteur (1) et lesdites surfaces, ainsi que tous les autres joints restant dans la zone entre des soudures adjacentes (3, 3'), sont hermétiques.

2. Combinaison selon la revendication 1, **caractérisée en ce que** la largeur (e) de ladite zone (4) est inférieure ou égale à 200 nm.

3. Combinaison selon la revendication 1 ou 2, **caractérisée en ce qu'**une deuxième soudure (5) a été formée de manière adjacente à une soudure (3).

4. Combinaison selon la revendication 1, 2 ou 3, **caractérisée en ce que** les extrémités d'une soudure (3) et/ou les extrémités d'une deuxième soudure (5) ont été agencées de manière à être essentiellement parallèles aux premier et deuxième bords (1.1, 1.2) du conducteur (1).

5. Combinaison selon l'une des revendications 1 à 4, **caractérisée en ce que** la couche conductrice (12) a essentiellement la même étendue que la première et/ou la deuxième couche(s) de substrat (10, 11).

6. Combinaison selon l'une des revendications 1 à 4, **caractérisée en ce que** la couche conductrice (12) a été mise en forme avant d'être ajustée en place de sorte que seules des parties formant les conducteurs (1) s'étendent jusqu'à la périphérie (21) de la pièce de substrat (P).

7. Combinaison selon l'une des revendications 1 à 6, **caractérisée en ce que** le matériau de la première et/ou de la deuxième couche(s) de substrat (10, 11) est du verre, du silicium, du saphir ou un matériau de combinaison de ceux-ci.

8. Combinaison selon l'une des revendications 1 à 7, **caractérisée en ce que** le matériau de la couche conductrice (12) et du conducteur (1) est l'ITO ou un composé métal/métal électriquement conducteur.

9. Procédé de mise en œuvre d'une traversée électrique hermétique dans une pièce de substrat (P), dans lequel ladite pièce est formée comme suit :
a. des première et deuxième couches de substrat (10, 11) sont placées l'une contre l'autre après l'ajustement d'un composant (13) dans un espace (20) entre lesdites couches de substrat et après le placement d'une couche conductrice (12) entre lesdites couches,
b. une traversée hermétique comprenant un premier conducteur (1) est ajustée entre les couches de substrat (10, 11), le premier conducteur (1) est formé de la couche conductrice (12), et dans lequel le premier conducteur (1) s'étend à partir de la périphérie (21) de la pièce de substrat (P) jusqu'au composant (13) pour fournir une connexion électrique depuis l'extérieur de la pièce de substrat (P) au composant (13),
**caractérisé en ce que**
c. les couches de substrat (10, 11) et la couche conductrice (12) ont une région d'interface commune (25), les couches de substrat (10, 11) et la couche conductrice (12) entre elles sont assemblées au moyen d'au moins une soudure (3, 3') réalisée avec un faisceau laser focalisé (14), dans lequel l'au moins une soudure (3, 3') entoure le composant (13) au niveau de la hauteur de la région d'interface commune (25), dans lequel chaque soudure s'étend essentiellement d'un premier bord (1.1) d'un conducteur (1) à son deuxième bord ou à un deuxième bord (1.2) d'un autre conducteur adjacent à celui-ci, de sorte que ladite traversée hermétique comprend une zone (4), qui est située entre le premier conducteur (1) et une soudure (3, 3'), dans lequel des joints situés dans ladite zone (4) entre les couches ne sont pas soudés ensemble,
d. des surfaces opposées des couches de substrat (10, 11) tournées vers le conducteur (1), une première surface (10.1) et une deuxième surface (11.2), sont resserrées sur le premier conducteur (1) après le soudage en raison de tensions thermiques provoquées par la chaleur de soudage, dans lequel des joints entre le premier conducteur (1) et lesdites surfaces, ainsi que tous les autres joints restant dans la zone entre des soudures adjacentes (3, 3'), sont hermétiques.

10. Procédé selon la revendication 9, **caractérisé en ce qu'**une deuxième soudure (5) est formée de manière adjacente à une première soudure (3), et dans lequel des extrémités desdites soudures (3, 5) sont essentiellement parallèles aux premier/deuxième bords (1.1, 1.2) du premier conducteur (1).
